# EUROPÄISCHE PATENTANMELDUNG

(11) **EP 1 314 444 A1**
(43) Veröffentlichungstag der Anmeldung: **28.05.2003**
(21) Anmeldenummer: 02026213.5
(22) Anmeldetag: 26.11.2002
(51) Int. Cl.: A61M 5/32, B02C 19/12

(54) **Zerkleinerungsvorrichtung für Kleingeräte und Verfahren zur Entsorgung von kontaminierten Kleingeräten**

(30) Priorität: 27.11.2001 DE 10158845
(71) Anmelder: SCHLEICHER & Co. INTERNATIONAL AKTIENGESELLSCHAFT, D-88677 Markdorf/Bodensee (DE)
(72) Erfinder: Schuster, Dieter, 88633 Hattenweiler (DE); Dieck, Leopold E., Dr., 88121 Ravensburg (DE)
(74) Vertreter: Patentanwälte , Ruff, Wilhelm, Beier, Dauster & Partner

(57) **Zusammenfassung**

Bei einer Zerkleinerungsvorrichtung für Kleingeräte mit einem Schneidwerk zum Zerkleinern wenigstens eines Teils des Kleingerätes in Zerkleinerungsgut und einem Sammelbehälter zum Auffangen des Zerkleinerungsgutes ist das Schneidwerk derart ausgebildet, dass das Kleingerät im wesentlichen vollständig durch einen selbsttätigen Zerkleinerungsvorgang auf einen Bruchteil seines ursprünglichen Volumens zerkleinerbar ist.

Bei einem Verfahren zur Entsorgung von kontaminierten Kleingeräten wird das Kleingerät zunächst in eine Zerkleinerungsvorrichtung, die ein Schneidwerk und einen Sammelbehälter für das Zerkleinerungsgut aufweist, zugeführt. Danach wird das Kleingerät im Schneidwerk auf einen Bruchteil seines ursprünglichen Volumens zerkleinert und das Zerkleinerungsgut in den Sammelbehälter transportiert. Das Zerkleinerungsgut wird entkeimt. Der Sammelbehälter wird vom Schneidwerk abgetrennt und versiegelt und kann entsorgt werden. Die Zerkleinerungsvorrichtung weist insbesondere ein Schneidwerk mit zwei gegenläufig rotierenden Schneidwalzen auf.

## Beschreibung

Die Erfindung betrifft eine Zerkleinerungsvorrichtung für Kleingeräte und ein Verfahren zur Entsorgung von kontaminierten Kleingeräten. Die Zerkleinerungsvorrichtung besitzt ein Schneidwerk, zum Zerkleinern wenigstens eines Teils des Kleingerätes in Zerkleinerungsgut und einen Sammelbehälter zum Sammeln des Zerkleinerungsgutes.

Es sind verschiedene Geräte entwickelt worden, um insbesondere medizinisches Kleingerät, vor allem Spritzen, zumindest teilweise zu vernichten. Aus der US 5 075 529 ist beispielsweise ein Verfahren bekannt, bei dem an eine Spritzennadel ein Magnetfeld gelegt wird und es infolge der Induktionswärme des Magnetfeldes zu einem Abschmelzen der Nadel kommt. Die abgeschmolzene Nadel fällt dann in einen dafür vorgesehenen Sammelbehälter und wird entsorgt.

Aus der US 4 275 628 ist ein Spritzennadelvernichter bekannt, der eine Öffnung besitzt, in die das Vorderteil der Spritze, der sog. "Hub", und die Spritzennadel einführbar sind. Der Hub ist der Verbindungsabschnitt zwischen der Spritzennadel und dem Korpus der Spritze und ist im Durchmesser wesentlich kleiner als der Spritzenkorpus. Durch das Niederdrücken eines Handgriffes wird ein Messer betätigt, das den Hub mit samt der Spritzennadel vom Rest der Spritze abschert. Der abgescherte Teil fällt in den Sammelbehälter des Spritzennadelvernichters und wird entsorgt.

Ein ähnliches Verfahren zur Vernichtung von Spritzennadeln aus der US 4 255 996 bekannt. Dort wird der Hub und die Nadel durch Verschwenken eines Schneidmessers (cutter) zwischen zwei Endpositionen abgeschert.

Aus der US 3 914 865 ist ein Handgerät zur Zerstörung von Spritzen bekannt. Dabei wird schrittweise durch wiederholtes Betätigen eines Handgriffs zunächst die Spritzennadel, dann der sogenannte Hub und dann der Rest der Spritze Stück für Stück durch ein Schneidmesser abgeschert. Die abgescherten Teile der Spritze fallen dann in einen dafür vorgesehenen Sammelraum des Handgerätes.

### Aufgabe und Lösung

Aufgabe der Erfindung ist es, eine Zerkleinerungsvorrichtung der eingangs erwähnten Art zu schaffen, mit dem Kleingeräte im Vergleich zum Stand der Technik schneller, kostengünstiger und sicherer zerkleinert werden können. Außerdem soll ein Verfahren geschaffen werden, mit dem kontaminierte Kleingeräte schnell und sicher entsorgt werden können.

Diese Aufgabe wird durch eine Zerkleinerungsvorrichtung mit den Merkmalen des unabhängigen Anspruches 1 und durch ein Verfahren mit den Merkmalen des unabhängigen Anspruches 12 gelöst. Weiterbildungen der Erfindung sind in den Unteransprüchen dargestellt und werden im Folgenden näher erläutert.

Die erfindungsgemäße Zerkleinerungsvorrichtung zeichnet sich dadurch aus, dass das Schneidwerk derart ausgebildet ist, dass das Kleingerät im wesentlichen vollständig durch einen selbsttätigen Zerkleinerungsvorgang auf einen Bruchteil seines ursprünglichen Volumens zerkleinerbar ist.

Im Gegensatz zu den meisten bekannten Zerkleinerungsvorrichtungen für Kleingeräte wird bei der erfindungsgemäßen das Kleingerät nahezu vollständig zerkleinert und im Sammelbehälter gesammelt. Es bleiben also keine Teile übrig, die möglicherweise getrennt entsorgt werden müssen. Kommt das Kleingerät in Kontakt mit dem Schneidwerk der Zerkleinerungsvorrichtung, beginnt ein selbsttätiger Zerkleinerungsvorgang, der gegebenenfalls durch leichtes Nachdrücken des Kleingerätes unterstützt werden kann. Im Gegensatz zur US 3,914,865 ist also kein wiederholtes aktives Betätigen eines Hebels oder dergleichen notwendig, sondern der Zerkleinerungsvorgang läuft von alleine ab. Dies spart Zeit.

Als Kleingeräte im Sinne der Anmeldung werden insbesondere Einmal- bzw. Wegwerf-Artikel aus dem medizinischen Bereich, aus Labors usw. verstanden. Dies sind beispielsweise Spritzen, Kanülen, Pipetten oder dergleichen. Die Kleingeräte können aus sprödem Material, wie beispielsweise Glas oder Metall oder aus weichem Material, wie Kunststoff oder dergleichen sein. Als Zerkleinern im Sinne der Anmeldung wird sowohl die Entstehung einzelner Partikel als auch ein Zerquetschen des Kleingerätes mit dem Entstehen einzelner größerer Bruchstücke verstanden. Einzelne, in der Regel gleichgroße Partikel entstehen dann, wenn sprödes Material, beispielsweise eine Spritze mit einer Spritzennadel aus Metall und einem Korpus aus Glas, zerkleinert wird. Bei weichen Materialien, wie Kunststoff, kommt es aber zu einem Zusammendrücken oder Quetschen, beispielsweise wenn Kunststoffspritzen zerkleinert werden.

Wie erwähnt beginnt mit dem Kontakt zwischen Kleingerät und Schneidwerk ein selbsttätiger Zerkleinerungsvorgang.

Besonders bevorzugt wird das Kleingerät dabei automatisch eingezogen. Es ist also kein Nachführen bzw. Nachdrücken per Hand mehr notwendig, sondern das Schneidwerk zieht das Kleingerät selbsttätig ein. Damit braucht die das Kleingerät zu führende Person nicht bei der Zerkleinerungsvorrichtung stehen bleiben, sondern kann sich zwischenzeitlich anderen Aufgaben widmen.

Im Schneidwerk wird das Kleingerät auf einen Bruchteil seines ursprünglichen Volumens zerkleinert, beispielsweise auf ca. ein Viertel bis ca. zwei Fünftel, insbesondere ein Drittel, seines ursprünglichen Volumens. Dabei werden Kleingeräte aus sprödem Material in der Regel auf ein kleineres Volumen zerkleinert als Kleingeräte aus weichem Material.

Das Schneidwerk kann aus einer Anordnung von Schneidmessern bestehen, die sich gegenläufig zueinander bewegen und eine Art Scherenbewegung ausführen, durch die das Kleingerät schrittweise abgeschert wird.

Bevorzugt ist jedoch, wenn das Schneidwerk aus gegenläufig rotierenden, in Schneideingriff stehenden Schneidwalzen besteht. Insbesondere einen sich zwei gegenläufig rotierende Schneidwalzen können eine Vielzahl von Schneidscheiben aufweisen, die in vorzugsweise in regelmäßigen Abständen an der Schneidwalze angeordnet sind. Der Schneideingriff kann dadurch erreicht werden, dass Schneidscheiben einer Schneidwalze in zwischen jeweils zwei Schneidscheiben der anderen Schneidwalze ausgebildete Zwischenräume eingreifen und umgekehrt.

Beim Zerkleinern der Kleingeräte werden relativ große Schneidkräfte benötigt. Dies wird insbesondere dadurch erreicht, dass der Durchmesser der Schneidscheiben größer ist als die Länge der Schneidwalzen. Beispielsweise kann das Verhältnis des Durchmessers der Schneidscheiben zur Schneidwalzenlänge ca. 0,5 bis 3, insbesondere 1,1 bis 1,7, betragen. Die relativ kurzen Schneidwalzen ermöglichen darüber hinaus eine relativ kompakte Bauweise des Schneidwerks und damit der gesamten Zerkleinerungsvorrichtung.

Bei einer Weiterbildung der Erfindung ist das Schneidwerk derart ausgebildet, dass damit kontaminierte Kleingeräte zerkleinert werden können. Kontaminierte Kleingeräte finden sich vor allem in Krankenhäusern usw.

Da das Schneidwerk in direktem Kontakt mit dem kontaminiertem Kleingerät steht, an dem oft noch Flüssigkeits- und Schmutzreste hängen, ist es insbesondere derart ausgebildet, dass eine Desinfizierung mit Desinfektionsflüssigkeit und/oder eine Sterilisierung mit Dampf möglich ist. Dies kann beispielsweise dadurch erfolgen, dass das Schneidwerk relativ einfach und schnell ausgebaut werden kann oder derart in der Zerkleinerungsvorrichtung angeordnet ist, dass es vollständig desinfizierbar bzw. sterilisierbar ist. Insbesondere sollten schwer zu desinfizierende Toträume vermieden werden, die potentielle Bakteriennester sind.
Um das Schneidwerk vor derartig aggressiven Medien zu schützen, kann es oberflächenvergütet sein. Das Schneidwerk, insbesondere die Schneidscheiben, können gehärtet sein, beispielsweise können die Schneidezähne der Schneidscheiben aus Hartmetall bestehen, um Abreibungseffekte zwischen dem spröden Material des Kleingerätes und den Schneidezähnen zu minimieren.

Durch die Flüssigkeits- oder Schmutzreste am Kleingerät sammelt sich im Sammelbehälter ein Sumpf aus diesen Rückständen an. Teilweise handelt es sich dabei um sehr aggressive Flüssigkeiten. Daher ist es notwendig, das Schneidwerk gegen Ausdünstungen solcher aggressiver Flüssigkeiten zu schützen. Dies wird insbesondere dadurch erreicht, dass das Schneidwerk gegenüber dem Sammelbehälter gekapselt, insbesondere über eine Dichtung abgedichtet ist.

Besonders bevorzugt besitzt die Zerkleinerungsvorrichtung eine Entkeimungseinrichtung zum Entkeimen von kontaminiertem Zerkleinerungsgut.

Die Entkeimung des zerkleinerten Kleingerätes erfolgt vorzugsweise durch UV-Bestrahlung. Es sind jedoch auch andere Entkeimungsarten denkbar. Vorzugsweise werden dabei UV-Strahler eingesetzt, die in Richtung des Zerkleinerungsgutes strahlen. Die UV-Strahler können im Bereich eines Deckelabschnittes der Zerkleinerungsvorrichtung angeordnet sein.

Die Zerkleinerungsvorrichtung ist bevorzugt in der Weise ausgebildet, dass beim Zuführen das Kleingerät im wesentlichen vollständig von der Zerkleinerungsvorrichtung umgeben ist.

Das Kleingerät gelangt vorzugsweise über einen Zuführkanal in das Schneidwerk der Zerkleinerungsvorrichtung. Der Zuführkanal ist dabei vorzugsweise derart ausgebildet, dass dessen Länge im wesentlichen der Länge des Kleingerätes entspricht. Dadurch wird erreicht, dass das Kleingerät im wesentlichen vollständig im Zuführkanal aufgenommen ist. Würde das Kleingerät ohne den entsprechenden Zuführkanal in die Zerkleinerungsvorrichtung gegeben werden, besteht die Gefahr, dass sich die Person, die das Kleingerät zuführt, an scharfkantigen Rändern oder sogar umherfliegenden Splittern, insbesondere bei Spritzen mit Glaskörpern, verletzen könnte. Durch den relativ langen Zuführkanal wird dies jedoch verhindert.

Die Erfindung umfasst ferner ein Verfahren zur Entsorgung von kontaminierten Kleingeräten mit den Merkmalen des unabhängigen Anspruches 16.

In Krankenhäusern, Kliniken, Arztpraxen usw. herrschen sehr hohe Anforderungen an die Sterilität der eingesetzten medizinischen Geräte. Deshalb haben sich insbesondere bei medizinischen Kleingeräten, wie Spritzen, Kanülen oder dgl., solche durchgesetzt, die nur einmal benutzt und dann weggeworfen werden. Dies führt natürlich zu einem immensen Anfall von einmal benutzten, kontaminierten, medizinischen Kleingeräten in den Krankenhäusern oder dgl., die entsorgt werden müssen. Dabei treten insbesondere zwei Entsorgungsstufen auf, nämlich die Erstentsorgung des benutzten Kleingerätes im Operationssaal oder dgl. und die Gesamtentsorgung der insgesamt anfallenden kontaminierten Abfälle. Oft werden diese Wegwerf-Kleingeräte erst einmal vor Ort gesammelt und dann irgendwann zur zentralen Entsorgungs- oder Vernichtungsstelle gebracht. Dies ist dann gefährlich, wenn das kontaminierte Kleingerät eine zeitlang in der Erstsammelstelle liegt, ohne dass es desinfiziert wird, da es dabei zu einem Ausdünsten von Keimen kommen kann, die die Sterilität im Raum beeinträchtigt. Das erfindungsgemäße Verfahren zeichnet sich dadurch aus, dass zunächst das kontaminierte Kleingerät einer Zerkleinerungsvorrichtung zugeführt wird. Die Zerkleinerungsvorrichtung besitzt ein Schneidwerk und einen Sammelbehälter für das Zerkleinerungsgut. Als nächsten Schritt wird das Kleingerät im Schneidgerät auf einen Bruchteil seines ursprünglichen Volumens zerkleinert. Danach wird das Zerkleinerungsgut in den Sammelbehälter transportiert. Danach wird eine Entkeimung des Zerkleinerungsgutes durchgeführt. Nach der Entkeimung wird der Sammelbehälter vom Schneidwerk abgetrennt und versiegelt. Der versiegelte Sammelbehälter kann dann als Sondermüll entsorgt werden. Die Zerkleinerungsvorrichtung stellt eine kompakte Einheit dar, die beispielsweise vor Ort in einem Operationssaal aufgestellt werden kann. Dadurch ist es möglich, das Kleingerät direkt nach dem Gebrauch vor Ort sicher zu entsorgen.

Die übliche Praxis in Labors, Krankenhäusern usw. ist oft diejenige, dass kontaminierte, nicht mehr brauchbare Kleingeräte zunächst einmal vor Ort in einen Sammelbehälter geworfen werden, der dann ab und zu entsorgt wird, d.h. zu einer zentralen Sammelstelle gebracht wird. Üblicherweise werden die in den Sammelbehälter geworfenen Kleingeräte zunächst nicht entkeimt. Dies birgt die Gefahr, dass Keime bzw. Bakterien in die Umgebung gelangen können. Besonders kritisch ist, dass in Operationssälen an die hohe Aseptik Anforderungen gestellt werden. Die andere Möglichkeit ist natürlich diejenige, die kontaminierten Kleingeräte sofort aus dem Aseptik-Bereich zur Sammelstelle zu bringen. Dies kostet wiederum Zeit. Das erfindungsgemäße Verfahren bietet nun die Möglichkeit, die kontaminierten Kleingeräte vor Ort sicher zu entsorgen und keimfrei zwischenzulagern.

Das Kleingerät kann derart zugeführt werden, dass es im wesentlichen vollständig von der Zerkleinerungsvorrichtung umgeben ist. Es kann automatisch vom Schneidwerk eingezogen werden. Durch das erfindungsgemäße Verfahren kann das Kleingerät auf ca. ein Viertel bis ca. zwei Fünftel, insbesondere ein Drittel, seines ursprünglichen Volumens zerkleinert werden. Das Zerkleinern kann im Schneidwerk durch zwei gegenläufig rotierende, in Schneideingriff stehende Schneidwalzen erfolgen. Die Entkeimung kann durch eine UV-Bestrahlung des Zerkleinerungsgutes erfolgen.

Die vorstehenden und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können, für die hier Schutz beansprucht wird. Die Unterteilung der Anmeldung in einzelne Abschnitte sowie Zwischenüberschriften beschränkt die unter diesen gemachten Aussagen nicht in ihrer Allgemeingültigkeit.

### Kurzbeschreibung der Zeichnung

Die erfindungsgemäße Zerkleinerungsvorrichtung ist in der Zeichnung dargestellt und wird im Folgenden näher erläutert. Die Zeichnung zeigt:

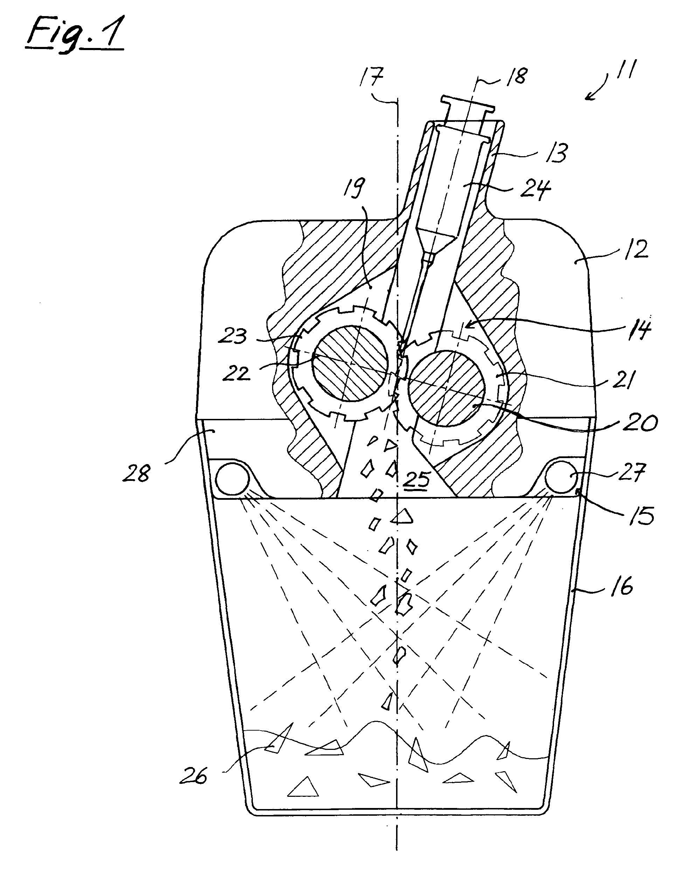

Fig. 1 einen Querschnitt durch die erfindungsgemäße Zerkleinerungsvorrichtung.

### Beschreibung des Ausführungsbeispiels und des Verfahrens

Die Figur zeigt die erfindungsgemäße Zerkleinerungsvorrichtung 11. Sie besteht im wesentlichen aus einem Deckelabschnitt 12 mit einem Zuführkanal 13, einem Schneidwerk 14 und einer Entkeimungsvorrichtung 15 sowie einem Sammelbehälter 16.

Wie in der Figur dargestellt, ragt ungefähr die Hälfte des Zuführkanales 13 von der Oberseite des Deckelabschnittes 12 ab. Die vertikale Mittelachse 17 durch die Zerkleinerungsvorrichtung 11 und die Mittelachse 18 durch den Zuführkanal liegen nicht parallel zueinander, sondern in einem Winkel ca. 10° bis 20°, insbesondere 15°. Der untere Teil des Zuführkanales 13 ist in den Deckelabschnitt 12 integriert. Der Zuführkanal 13 erweitert sich an diesem Ende trichterförmig zu einem Schneidraum 19, in dem sich das Schneidwerk 14 befindet. Die Gesamtlänge des Zuführkanals 13 entspricht im wesentlichen der Länge des zugeführten Kleingerätes 24. Der Zuführkanal 13 hat vorzugsweise einen kreisförmigen Querschnitt. Es sind jedoch auch andere Querschnittsformen möglich.

Das Schneidwerk 14 besteht im wesentlichen aus zwei gegenläufig rotierenden Schneidwalzen 20, die durch einen Motor (nicht dargestellt), der sich ebenfalls im Deckelabschnitt 12 befindet, angetrieben werden. An den Schneidwalzen 20 befinden sich jeweils eine Reihe von Schneidscheiben 21, von denen im beschriebenen Ausführungsbeispiel repräsentativ jeweils eine Schneidscheibe im Schnitt dargestellt ist. Die Schneidwalzen 20 sind versetzt zueinander angeordnet, so dass die Schneidscheiben 21 der einen Schneidwalze in die zwischen zwei Schneidscheiben ausgebildeten Zwischenräume der anderen Schneidwalze 20 eingreifen und umgekehrt. Dadurch wird ein Schneideingriff gebildet. Wie in der Figur dargestellt, sind die vertikalen Mittelachsen 22 der Schneidwalzen ebenfalls im Winkel von ca. 10 bis 20° zur vertikalen Mittelachse der Zerkleinerungsvorrichtung 11 angeordnet. Am Umfang der Schneidscheiben 21 befinden sich, in regelmäßigen Abständen angeordnet, Schneidezähne 23, die die Zerkleinerung des zugeführten Kleingerätes 24 bewirken. Ist das zugeführte Kleingerät 24, wie im dargestellten Ausführungsbeispiel eine Spritze mit Glaskörper, wird durch die Schneidezähne ein Partikelschnitt erzeugt. Das Kleingerät 24 wird also in einzelne Partikeln zerkleinert.

Da beim Zerkleinern solcher Kleingeräte 24 relativ große Schneidkräfte aufgebracht werden müssen, besitzt das Schneidwerk 14 eine besondere Schneidgeometrie. Dabei ist der Durchmesser der Schneidscheiben 21 größer als die Länge der Schneidwalzen 20. Das Verhältnis des Durchmessers der Schneidscheiben 21 zur Schneidwalzenlänge beträgt insbesondere 1,1 bis 1,7. Somit entsteht ein relativ kompaktes Schneidwerk 14. Durch die relativ großen Durchmesser der Schneidscheiben 21 wird ein hohes Drehmoment erzeugt. Unterhalb des Schneidwerks 14 befindet sich ein kurzer Transportkanal 25, über den das Zerkleinerungsgut 26, also das zerkleinerte Kleingerät 24, in den Sammelbehälter 16 transportiert wird. Der Schneidraum 19 verengt sich unterhalb des Schneidwerkes 14 trichterförmig. Das Schneidwerk 14 ist gegenüber dem Sammelbehälter 16 und gegenüber seinem Antrieb gekapselt. Ferner ist der Schneidraum 19, der das Schneidwerk 14 umgibt, derart ausgebildet, dass das Schneidwerk einfach desinfiziert oder sterilisiert werden kann. Vor allem befinden sich im Schneidraum 19 keine Toträume, die potentielle Bakterien oder Keimnester bilden können. Um das Schneidwerk 14, insbesondere die Schneidscheiben 21 vor den aggressiven Medien, beispielsweise der Desinfizierflüssigkeit oder der Flüssigkeitsreste an den Kleingeräten zu schützen. sind die Schneidscheibenoberflächen vergütet, vorzugsweise gehärtet und/oder beschichtet.

Im unteren Bereich des Mittelabschnittes 12 befindet sich die Entkeimungseinrichtung 15, die im beschriebenen Ausführungsbeispiel aus zwei UV-Strahlern 27 besteht, die zum Sammelbehälter 16, insbesondere zum Boden des Sammelbehälters 16, hin ausgerichtet sind.

Der Sammelbehälter 16 ist lösbar am Deckelabschnitt 12 der Zerkleinerungsvorrichtung 11 befestigt. Die Befestigung des Sammelbehälters 16 kann beispielsweise, wie im beschriebenen Ausführungsbeispiel dargestellt, durch Aufstecken auf den Deckelabschnitt 12 der Zerkleinerungsvorrichtung, insbesondere auf einen dafür ausgebildeten Steckabschnitt 28, erfolgen. Es sind jedoch auch andere Befestigungsmöglichkeiten von Sammelbehälter 16 und Deckelabschnitt 12 denkbar, beispielsweise mittels einer Schraub-, Klemm-Verbindung oder dergleichen.

Das Verfahren zur Entsorgung eines kontaminierten Kleingerätes 24 und die Funktion der Zerkleinerungsvorrichtung 11 sollen beispielhaft anhand der Zerkleinerung einer Spritze mit Glaskörper beschrieben werden. Zunächst wird die kontaminierte Spritze an den Zuführkanal 13 herangeführt. Danach wird die Spritze in den Zuführkanal 13 gesteckt, wobei die Spritzennadel in Kontakt mit dem Schneidwerk 14 kommt.

Durch den relativ langen Zuführkanal 13 ist die Spritze fast vollständig darin aufgenommen, so dass sich der Zuführende nicht an scharfkantigen Rändern oder umherfliegenden Splittern verletzen kann. Dadurch, dass die Schneidezähne 23 die Spritzennadel greifen, wird die gesamte Spritze automatisch, also selbsttätig eingezogen und Stück für Stück zerkleinert. Im Falle der metallenen Spritzennadel und dem Korpus der Spritze aus Glas erzeugt das Schneidwerk einen Partikelschnitt. Bei einem Kleingerät aus Kunststoff kommt es zu einem anderen Zerkleinerungsvorgang. Das Kunststoff-Kleingerät wird dabei nämlich zunächst zusammengequetscht und nicht in einzelne relativ kleine Partikel zerkleinert, sondern in einzelne relativ große Stücke. Das Zerkleinerungsgut 26, im beschrienen Ausführungsbeispiel also die zerkleinerte Spritze, fällt über den Transportkanal 25 auf den Boden des Sammelbehälters 16. Die Partikel der Spritze und die Flüssigkeitsreste, die an der kontaminierten Spritze hängen geblieben sind, sammeln sich als Sumpf am Boden des Sammelbehälters 16 an.

Als nächstes werden die Spritzenpartikel durch die Entkeimungsvorrichtung 15, die sich am Deckelabschnitt 12 befindet und in Richtung Sammelbehälter 16 ausgerichtet ist, entkeimt. Die Entkeimung kann auf zwei verschiedene Arten erfolgen. Die Entkeimungsvorrichtung 15, die im beschriebenen Ausführungsbeispiel aus zwei UV-Strahlen 27 besteht, kann permanent in den Sammelbehälter strahlen und so eine Entkeimung bewirken. Eine andere Möglichkeit ist, dass nach dem Zerkleinern einer Charge, beispielsweise der Spritze, die Entkeimungsvorrichtung eingeschaltet wird und eine zeitlang entkeimt wird. Hat der Sammelbehälter 16 einen gewissen Füllstand an Zerkleinerungsgut 26 erreicht, muss er ausgewechselt werden. Dazu wird er vom Deckelabschnitt 12 abgetrennt, indem er von dem Steckabschnitt 28 des Deckelabschnittes 12 abgezogen wird. Als nächstes wird der abgezogene Sammelbehälter 16 versiegelt, beispielsweise mit einem geeigneten Deckel.

Um das Schneidwerk 14 zu desinfizieren, wird die Desinfizierflüssigkeit in den Zuführkanal 13 gegeben, von wo sie in den Schneidraum 19 und an das zu desinfizierende Schneidwerk 14 gelangt. Die Desinfizierflüssigkeit sammelt sich dann im Sammelbehälter 16 an und kann dann in gleicher Weise wie das Zerkleinerungsgut 26 entsorgt werden.

## Patentansprüche

1. Zerkleinerungsvorrichtung für Kleingeräte, insbesondere Spritzen oder dergleichen mit einem Schneidwerk zum Zerkleinern wenigstens eines Teils des Kleingerätes in Zerkleinerungsgut und einem Sammelbehälter zum Auffangen des Zerkleinerungsgutes, **dadurch gekennzeichnet, dass** das Schneidwerk (14) derart ausgebildet ist, dass das Kleingerät (24) im Wesentlichen vollständig durch einen selbsttätigen Zerkleinerungsvorgang auf einen Bruchteil seines ursprünglichen Volumens zerkleinerbar ist.

2. Zerkleinerungsvorrichtung nach Anspruch 1, **dadurch gekennzeichnet, dass** das Schneidwerk (14) derart ausgebildet ist, dass das Kleingerät (24) automatisch einziehbar ist.

3. Zerkleinerungsvorrichtung nach einem der Ansprüche 1 oder 2, **dadurch gekennzeichnet, dass** das Schneidwerk (14) derart ausgebildet ist, dass das Kleingerät (24) auf ca. ein Viertel bis ca. zwei Fünftel, insbesondere ein Drittel, seines ursprünglichen Volumens zerkleinerbar ist.

4. Zerkleinerungsvorrichtung nach einem der vorhergehenden Ansprüche, **dadurch gekennzeichnet, dass** das Schneidwerk (14) gegenläufig rotierende, in Schneideingriff stehende Schneidwalzen (20) aufweist, wobei vorzugsweise die Schneidwalzen (20) eine Vielzahl von Schneidscheiben (21) aufweisen und wobei die Schneidscheiben (21) einer Schneidwalze (20) in komplementär dazu ausgebildete Zwischenräume zwischen jeweils zwei Schneidscheiben (21) der anderen Schneidwalze (20) eingreifen und umgekehrt.

5. Zerkleinerungsvorrichtung nach Anspruch 4, **dadurch gekennzeichnet, dass** das Verhältnis des Durchmessers der Schneidscheiben (21) zur Schneidwalzenlänge ca. 0,5 bis 3, insbesondere 1,1 bis 1,7 beträgt.

6. Zerkleinerungsvorrichtung nach einem der vorhergehenden Ansprüche, **dadurch gekennzeichnet, dass** das Schneidwerk derart ausgebildet ist, dass kontaminierte Kleingeräte (24) zerkleinerbar sind, wobei vorzugsweise das Schneidwerk (14) desinfizierbar und/oder sterilisierbar ist.

7. Zerkleinerungsvorrichtung nach einem der vorhergehenden Ansprüche, **dadurch gekennzeichnet, dass** das Schneidwerk (14), insbesondere die Schneidscheiben (21), oberflächenvergütet, vorzugsweise gehärtet und/oder beschichtet sind.

8. Zerkleinerungsvorrichtung nach einem der vorhergehenden Ansprüche, **dadurch gekennzeichnet, dass** das Schneidwerk (14) gegenüber dem Sammelbehälter (16) gekapselt ist, insbesondere über eine Dichtung abgedichtet ist.

9. Zerkleinerungsvorrichtung nach einem der vorhergehenden Ansprüche, **dadurch gekennzeichnet, dass** sie eine Entkeimungseinrichtung (15) zum Entkeimen von kontaminiertem Zerkleinerungsgut (26) aufweist, insbesondere eine UV-Strahlungseinrichtung, wobei vorzugsweise die Entkeimungseinrichtung (15) in Richtung Boden des Sammelbehälters (16) ausgerichtet und insbesondere an einem Deckelabschnitt (12) der Zerkleinerungsvorrichtung (11) angeordnet ist.

10. Zerkleinerungsvorrichtung nach Anspruch 9, **dadurch gekennzeichnet, dass** der Sammelbehälter (16) lösbar am Deckelabschnitt (12) befestigbar, insbesondere auf den Deckelabschnitt (12) aufsteckbar ist.

11. Zerkleinerungsvorrichtung nach einem der vorhergehenden Ansprüche, **dadurch gekennzeichnet, dass** sie derart ausgebildet ist, dass das Kleingerät (24) beim Zuführen im wesentlichen vollständig von der Zerkleinerungsvorrichtung umgeben ist, vorzugsweise einen Zuführkanal (13) aufweist, dessen Länge im wesentlichen der Länge des Kleingerätes entspricht.

12. Verfahren zur Entsorgung von kontaminierten, insbesondere medizinischen, Kleingeräten mit folgenden Schritten:
- Zuführen des Kleingerätes in eine Zerkleinerungsvorrichtung, die ein Schneidwerk und einen Sammelbehälter für Zerkleinerungsgut aufweist;
- Zerkleinern des Kleingerätes im Schneidwerk auf ein Bruchteil seines ursprünglichen Volumens und Transport des Zerkleinerungsgutes in den Sammelbehälter;
- Entkeimung des Zerkleinerungsgutes und
- Abtrennen des Sammelbehälters vom Schneidwerk und Versiegeln des Sammelbehälters.
